(19) Europäisches Patentamt
European Patent Office
Office européen des brevets

(11) Publication number: 0 399 842 A2

# (12) EUROPEAN PATENT APPLICATION

(21) Application number: 90305730.5

(22) Date of filing: 25.05.90

(51) Int. Cl.5: A61K 31/435, A61K 31/025, A61K 9/107

(30) Priority: 26.05.89 JP 133948/89

(43) Date of publication of application: 28.11.90 Bulletin 90/48

(84) Designated Contracting States: BE CH DE DK ES FR GB IT LI NL SE

(71) Applicant: THE GREEN CROSS CORPORATION
3-3, Imabashi 1-chome Chuo-ku
Osaka-shi Osaka(JP)

(72) Inventor: Tsuda, Yoshio, c/o Chuo Kenkyusho
The Green Cross Corp., 1180-1,
Shodaiotani-2-chome
Hirakata-shi(JP)
Inventor: Murashima, Ryoichiro, c/o Chuo Kenkyusho
The Green Cross Corp., 1180-1,
Shodaiotani-2-chome
Hirakata-shi(JP)
Inventor: Yamanouchi, Kouichi, c/o Chuo Kenkyusho
The Green Cross Corp., 1180-1,
Shodaiotani-2-chome
Hirakata-shi(JP)
Inventor: Yokoyama, Kazumasa, c/o Chuo Kenkyusho
The Green Cross Corp., 1180-1,
Shodaiotani-2-chome
Hirakata-shi(JP)

(74) Representative: Coleiro, Raymond et al
MEWBURN ELLIS 2 Cursitor Street
London EC4A 1BQ(GB)

## (54) Emulsified preparations containing perfluorocarbon compound.

(57) An emulsified preparation containing a perfluorocarbon compound comprises 5-50% (w/v) of a perfluorocarbon compound represented by the formula:

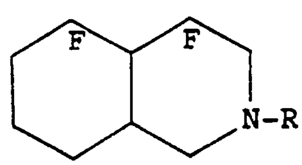

(wherein R represents a lower perfluoroalkyl group), 1-5% (w/v) of a phospholipid, 0.01-0.1% (w/v) of a fatty acid or an ester or a salt thereof, 0.001-0.01% (w/v) of tocopherol, 0.011-1.1% (w/v) of a pH adjuster, and an adequate amount of an aqueous solvent. This emulsified preparation can be used as an oxygen-carrying infusion fluid or perfusion fluid for preserving organs.

EP 0 399 842 A2

# EMULSIFIED PREPARATIONS CONTAINING PERFLUOROCARBON COMPOUND

## BACKGROUND OF THE INVENTION

This invention relates to emulsified preparations (which may be simply called emulsions) containing a perfluorocarbon compound which are useful, for instance, as an oxygen-carrying infusion fluid (so-called red blood cell substitute) or a perfusion fluid for preserving living organs.

It has already been reported that fluorocarbon emulsions have the potential for use as a red blood cell substitute for mammals and an organ-preservative perfusion fluid used in the event of transplantation of organs, especially as an infusion fluid capable of functioning as oxygen carrier. (See, for example, Clark C. Leland, Jr., F. Ecattini and S. Kaplan: The Physiology of Synthetic Blood, J. Thoracic Cardiovascular Surg., Vol. 60, pp. 757-773, 1970; and R.P. Geyer: Fluorocarbon-polyol Artificial Blood Substitutes, New Engl. J. Med., Vol. 289, pp. 1077-1082, 1973).

The conventional fluorocarbon emulsions, however, can hardly be rated to be practical because of their pharmacological instability. For realizing practical utilization of fluorocarbon emulsions as artificial erythrocytes, it is necessary to develop a preparation which is stable and remains unchanged in particle size for a long time.

In fluorocarbon emulsions, the particle size thereof is a key factor for the toxicity and efficacy of the emulsion. (See, for example, K. Yokoyama, K. Yamanouchi, M. Watanabe, R. Murashima, T. Matsumoto, T. Hamano, H. Okamoto, T. Suyama, R. Watanabe and R. Naito: Preparation of Perfluorodecalin Emulsion, an Approach to the Red Cells Substitute, Federation Proceeding, Vol. 34, pp. 1478-1483. May, 1975). A fluorocarbon emulsion having a large particle size is high in toxicity and also short in retention time of particles in the blood flow. Therefore, when a fluorocarbon emulsion is used as a lifesaving infusion of artificial erythrocytes for a patient suffering from profuse bleeding, such an emulsion should have an average particle size of less than 0.3 μ, preferably less than 0.2 μ (U.S. Patent 3,958,014).

Apart from particle size, in case of using a fluorocarbon as artificial red cells to be administered intravenously, it is required that the fluorocarbon be quickly egested out of the body after it has completed its normal function of oxygen carriage.

The present inventors had synthesized the perfluoro compounds represented by the formula:

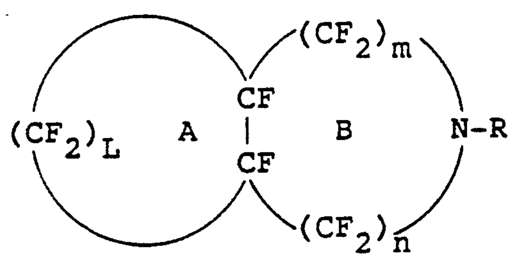

wherein one or both of the ring A and the ring B may be substituted with a lower perfluoroalkyl group; ℓ is a number of 3 or 4; m and n are the numbers that make 2 or 3 when added together; and R represents a lower perfluoroalkyl group (U.S. Patent 4,713,459), and found that the specific ones of these compounds, viz. the novel perfluoro compounds represented by the formula:

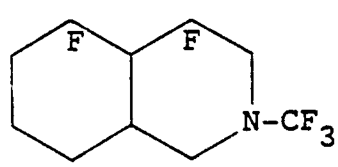

were capable of producing an emulsion of fine particles having long-time stability and could also be easily egested out of the living body (U.S. Patent 4,591,593).

Based on these findings, the present inventors have made further researches on the emulsified preparations containing the perfluorocarbon compounds having specific structures and, as a result, found that the desired preparations can be obtained by using the above-specified compounds or their analogs in

combination with other adequate substances including new components other than those used in the above emulsion for forming a specific composition, and this finding had led to the attainment of the present invention.

## SUMMARY OF THE INVENTION


The emulsified preparations according to this invention comprise 5 to 50% (w/v) of a perfluorocarbon compound represented by the formula (I):

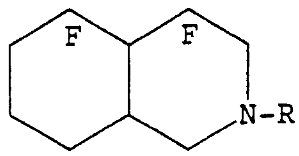

(I)

(wherein R represents a lower perfluoroalkyl group), 1 to 5% (w/v) of phospholipid, 0.01 to 0.1% (w/v) of a fatty acid or an ester or salt thereof, 0.001 to 0.01% (w/v) of tocopherol, 0.011 to 1.1% (w/v) of a pH adjuster and an appropriate amount of an aqueous solvent.


## DESCRIPTION OF THE PREFERRED EMBODIMENTS

The present invention will be described in further detail below.

The emulsified preparations provided according to this invention are oil-in-water type emulsions having a perfluorocarbon compound (I) dispersed in water.

### (1) Composition

#### (i) Perfluorocarbon compounds (I)

The lower perfluoroalkyl groups represented by R in the formula (I) may be of either straight-chain or branched structure and have 1 to 4, preferably 1 to 2 carbon atoms. Examples of such lower perfluoroalkyl groups include the perfluoromethyl group, perfluoroethyl group, perfluoro-n-propyl group, perfluoro-iso-propyl group, perfluoro-n-butyl group, perfluoro-iso-butyl group, perfluoro-sec-butyl group and the perfluoro-tert-butyl group.

The perfluorocarbon compounds (I) can be prepared by known methods (such as shown in U.S. Patent 4,713,658). That is, the compounds (I) can be produced by fluorinating perhydro compounds corresponding to the compounds (I). Fluorination in the above preparation can be effected by various methods such as a direct fluorination method, cobalt fluorination method or electrolytic fluorination method.

The content of perfluorocarbon compound (I) in the emulsified preparations in this invention is 5 to 50% (w/v), preferably 10 to 40% (w/v).

#### (ii) Phospholipid

In the present invention, phospholipid is used as an emulsifier for preparing the emulsion.

Preferred examples of phospholipid used in this invention are egg yolk phospholipid,and soybean phospholipid.

The content of phospholipid in the emulsified preparations is 1 to 5% (w/v), preferably 2.0 to 3.5% (w/v).

#### (iii) Fatty acid or an ester or salt thereof

In the emulsion preparation of this invention, a fatty acid or an ester or salt thereof is used as an auxiliary emulsifier.

The fatty acid or an ester or salt thereof used in the present invention is usually one having 8 to 22, preferably 14 to 20 carbon atoms. Salts of fatty acids usable in this invention are not subject to any specific limitation as far as they are pharmaceutically acceptable ones. Typical examples of such salts are alkali metal salts (sodium salt, potassium salt, etc.). Exemplary of the fatty acid esters usable in this invention are alkyl esters and monoglycerides.

Specific examples of these compounds are caprylic acid, capric acid, lauric acid, myristic acid, palmitic acid, stearic acid, behenic acid, palmitoleic acid, oleic acid, linolic acid, arachidonic acid, their sodium or potassium salts, and their monoglycerides.

Among the above compounds, oleic acid, linoleic acid, or their sodium or potassium salt are preferable. The content of fatty acid or ester or salt thereof in the emulsified preparation is 0.01 to 0.1% (w/v), preferably 0.02 to 0.05% (w/v).

(iv) Tocopherol

In the emulsified preparations of this invention, tocopherol is used as an antioxidant or stabilizing agent. Tocopherol includes α-, β-, γ- or δ-tocopherol, and may be isomers, such as, dℓ-form, ℓ-form and d-form.

The content of tocopherol in the emulsified preparation is 0.001 to 0.01% (w/v), preferably 0.002 to 0.005% (w/v).

(v) pH adjuster

In the emulsified preparations according to this invention, one or more substances may be used as a pH adjuster so long as they have buffer action. As the pH adjuster there may be used phosphates, citrates, acetates, tris(oxymethyl)aminomethane and the like in the form of buffer solution, among which phosphates is preferable. In the case of phosphates, dihydrogenphosphates and hydrogenphosphates may be used at the same time as a pH adjuster.

The content of the pH adjuster in the emulsified preparation is 0.011 to 1.1% (w/v), preferably 0.035 to 0.24% (w/v).

Examples of the dihydrogenphosphates usable in this invention are sodium dihydrogenphosphate and potassium dihydrogenphosphate.

The content of dihydrogenphosphate in the emulsified preparation is preferably 0.001 to 0.1% (w/v), more preferably 0.005 to 0.04% (w/v).

Examples of the hydrogenphosphates usable in this invention are sodium hydrogenphosphate and potassium hydrogenphosphate.

The content of hydrogenphosphate in the emulsified preparation is preferably 0.01 to 1.0% (w/v), more preferably 0.03 to 0.2% (w/v).

(vi) Aqueous solvent

In the emulsified preparations of this invention, various types of aqueous solvent can be used as far as they are pharmaceutically acceptable ones. Typical examples of such aqueous solvents are distilled water for injection, sterile water, saccharide solutions, and electrolyte, among which, distilled water is preferable.

(2) Process for producing emulsified preparation

The emulsified preparations of this invention can be produced by mixing the component substances in an optical order to form a crude emulsion and then homogenizing it by treating with a suitable emulsifier (e.g., injection type emulsifier mfd. by Manton-Gauling Co.) so that the particle size of the emulsion will become 0.05 to 0.3 µm, preferably 0.05 to 0.25 µm (U.S. Patent 4,591,593).

This can be accomplished, for instance, in the following way.

Initially phospholipid is added to an aqueous solvent and they are mixed up by stirring with a mixer, biotron, propeller agitator or the like. Then a perfluorocarbon compound is added to the mixture while

continuing its stirring, after which the necessary additives are added and the mixture is emulsified by an emulsifier. Alternatively, the component substances are mixed and stirred by a mixer or other means to form a crude emulsion and this crude emulsion is homogenized by an emulsifier.

The prepared emulsion may be subjected to an additional operation such as centrifugation for uniformalizing the particle size distribution.

(3) Uses

The emulsified preparations of this invention, as they have an oxygen carrying ability, can be used as an oxygen-carrying infusion fluid (so-called red cell substitute), perfusion fluid for preserving organs, and the like.

The present preparations are also useful for the improvement of myocardial infraction, activation of cerebral blood flow and treatment of burns. Further, they can be effectively utilized for percutaneous transluminal coronary angioglasty (PCTA) and combined therapy with blood vessel contrast medium, radiotherapic or chemotherapic agents for cancer, etc.

When the emulsified preparation of this invention is used as an oxygen-carrying infusion fluid for instance, it is usually administered by intravenous injection at a single dose of about 50 to 2,000 ml for adults.

When used for PTCA, the preparation is administered by arterial injection at a single dose of about 5 to 500 ml for adults.

The emulsified preparations of this invention are small in particle size, have excellent storage stability and can be easily egested out of the body.

Further, the preparations of this invention are egested quicker than a similar commercial preparation FLUOSOL-DA (composed principally of perfluorodecaline and perfluorotripropylamine, mfd. by The Green Cross Corporation, U.S. Patent 4,252,827) and can be stored under refrigeration.

Thus, the emulsified preparations of this invention can be highly rated in practical utility as final preparations.

The present invention will hereinafter be described more particularly with reference to the following Examples and Reference Examples, which, however, are merely illustrative and do not limit the scope of the invention.

Referential Example 1

This was carried out using a monel metal 1.5-litre electrolytic cell having therein the nickel-made plates (purity: over 99.6%) (6 anode plates and 7 cathode plates) arranged alternately with a plate-to-plate spacing of 1.7-2.0 mm and an effective anode area of 10.5 $dm^2$ and provided with a copper reflux condenser at the top of the cell.

1.2 litre of hydrogen fluoride was supplied into the electrolytic cell and trace amounts of impurities (water and sulfuric acid) were removed by preliminary electrolysis. Then 0.85 mol (130 g) of N-methyldecahydroisoquinoline was dissolved in hydrogen fluoride, and electrolysis was carried out at an anode current density of 1.0-2.0 $A/dm^2$, a voltage of 4.0-6.2 V and a bath temperature of 4-10 °C while passing helium gas into the cell from its bottom at a flow rate of 100 ml/min. 1,051 A•hr electrolysis was continued until the electrolytic voltage reached 9.0 V. Hydrogen fluoride was replenished at a rate of 200 ml per 24 hours. The gas produced during electrolysis was first passed through an iron tube packed with sodium fluoride pellets to remove accompanying hydrogen fluoride and then led into a trap cooled with dry ice/acetone mixture where the gas was liquefied and collected. 9.5 g of a colorless liquid was obtained. Meantime the bath liquid in the electrolytic cell divided into two layers, the upper layer composed of hydrogen fluoride and the lower layer composed of fluorocarbons. The lower layer was separated to obtain 263 g of liquid.

The liquid obtained by liquefying said gas was joined with the liquid forming the lower layer of electrolyte, and the mixed solution was added with an equal volume of a 70% aqueous solution of potassium hydroxide and diisobutylamine and refluxed for 7 days. The perfluoro substance was separated from the mixture by a separating funnel, washed with a 90 w/v % aqueous solution of acetone containing 10 w/v % of potassium iodide and then subjected to precision frationation by a precision fractionating apparatus having a spinning band column to obtain 44 g of perfluoro-N-methyldecahydroisoquinoline (yield: 10%; boiling point: 151-155 °C/760 mmHg).

The results of analyses of the obtained compound by IR absorption spectrum, F nuclear magnetic resonance spectrum and mass spectrum identified it as the objective compound perfluoro-N-methyldecahydroisoquinoline.

Referential Example 2

In order to show the effect of tocopherol and pH adjusters used in this invention in the storage stability, the following comparative experiment was conducted:

(1) Preparation of emulsion

The emulsions shown in Table 1 were prepared in the same manner as in Example 1 shown later.

Table 1

| | Emulsion | |
|---|---|---|
| | Present invention | Comparison* |
| Perfluoro-N-methyldecahydroisoquinoline | 25% (w/v) | 25% (w/v) |
| Egg yolk phospholipid | 3% (w/v) | 3% (w/v) |
| Tocopherol | 0.003% (w/v) | - |
| $NaH_2PO_4$ | 0.012% (w/v) | - |
| $Na_2HPO_4$ | 0.056% (w/v) | - |

*: Main components of U.S. Patent 4,591,593.

(2) Method of Experiment

As an indication of stabilizing effect, a change of pH value and an amount of free fatty acid formed during the storage were determined. After preparing each emulsion, they were subjected to sterilization treatment at 121°C for 5 minutes, and then storaged at 40°C for 3 months, and sterilization effect was observed in each case. pH was determined by a pH meter and the amount of free fatty acid formed was determined by a neutralization titration method. The results obtained were shown in Tables 2 and 3.

Table 2

| | pH | |
|---|---|---|
| | Before treatment | After storage |
| Present invention | 7.4 | 7.1 |
| Comparison | 7.4 | 5.8 |

The decrease of pH value was inhibited by the use of tocopherol and pH adjusters.

Table 3

| | Amount of free fatty acid formed (mEq/l) | |
|---|---|---|
| | Before treatment | After storage |
| Present invention | 0.5> | 2.0 |
| Comparison | 0.5> | 2.7 |

The increase of free fatty acid was suppressed by the use of tocopherol and pH adjusters.

The inhibition of decrease of pH value and the suppression of free fatty acid formation during the storage of the emulsified preparation containing a perfluorocarbon compound of this invention as mentioned above indicate the excellent stability of the emulsified preparation.

Example 1

6 kg of egg yolk phospholipid, 80 g of potassium oleate and 6 g of tocopherol were added to 160 litres of distilled water for injection and stirred in a mixer to prepare a crude emulsion. Then 50 kg of perfluoro-N-methyldecahydroisoquinoline was added, and after stirring by a mixer, 5.2 litres of a phosphate buffer solution was added, followed by further stirring. The resulting crude emulsion was put into a liquid tank in an injection type emulsifier (mfd. by Manton-Gauling Co.) and circulated for effectuating emulsification in a nitrogen gas stream under pressure of 100-600 kg/cm$^2$ while maintaining the liquid temperature at 65-70 °C.

The emulsified preparation thus obtained was charged into a vial for injection. After atmospheric replacement with nitrogen gas, the vial was stoppered and its content was sterilized by heating. The emulsified preparation thus obtained was stored in a cold place.

The particle size of the preparation measured by the light-scattering method was 0.2 µm.

Example 2

A perfluorocarbon-based pharmaceutical emulsified preparation of the following composition was prepared.

| | |
|---|---|
| Perfluoro-N-methyldecahydroisoquinoline | 25% (w/v) |
| Egg yolk phospholipid | 3% (w/v) |
| Potassium oleate | 0.04% (w/v) |
| Tocopherol | 0.003% (w/v) |
| Sodium dihydrogenphosphate•$2H_2O$ | 0.0115% (w/v) |
| Sodium hydrogenphosphate•$12H_2O$ | 0.14% (w/v) |
| Distilled water for injection | adequate amount |

Example 3

A perfluorocarbon-based pharmaceutical emulsified preparation of the following composition was prepared.

| | |
|---|---|
| Perfluoro-N-methyldecahydroisoquinoline | 20% (w/v) |
| Egg yolk phospholipid | 2.4% (w/v) |
| Oleic acid | 0.028% (w/v) |
| Tocopherol | 0.0024% (w/v) |
| Sodium dihydrogenphosphate | 0.096% (w/v) |
| Sodium hydrogenphosphate | 0.045% (w/v) |
| Sodium chloride | 0.6% (w/v) |
| Potassium chloride | 0.034% (w/v) |
| Calcium chloride | 0.028% (w/v) |
| Magnesium chloride | 0.02% (w/v) |
| Sodium hydrogencarbonate | 0.21% (w/v) |
| Glucose | 0.21% (w/v) |
| Distilled water for injection | adequate amount |

## Claims

1. An emulsified preparation containing a perfluorocarbon compound, comprising 5-50% (w/v) of a perfluorocarbon compound represented by the formula:

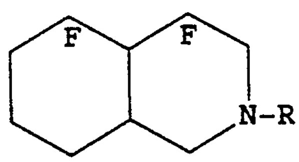

(wherein R represents a lower perfluoroalkyl group), 1-5% (w/v) of a phospholipid, 0.01-0.1% (w/v) of a fatty acid or an ester or a salt thereof, 0.001-0.01% (w/v) of tocopherol, 0.011-1.1% (w/v) of a pH adjuster and an adequate amount of an aqueous solvent.

2. An emulsified preparation according to Claim 1, wherein the lower perfluoroalkyl group is a perfluoromethyl group, perfluoroethyl group, perfluoro-n-propyl group, perfluoro-iso-propyl group, perfluoro-n-butyl group, perfluoro-iso-butyl group, perfluoro-sec-butyl group, or perfluoro-tert-butyl group.

3. An emulsified preparation according to Claim 1 or 2, wherein the phospholipid is egg yolk phospholipid or soybean phospholipid.

4. An emulsified preparation according to Claim 1, 2 or 3, wherein the fatty acid or an ester or a salt thereof is caprylic acid, capric acid, lauric acid, myristic acid, palmitic acid, stearic acid, behenic acid, palmitoleic acid, oleic acid, linolic acid, arachidonic acid, or a sodium or potassium salt, or a monoglyceride thereof.

5. An emulsified preparation according to any preceding Claim, wherein the pH adjuster is a phosphate, citrate, acetate, or tris(oxymethyl)aminomethane.

6. An emulsified preparation according to Claim 5, wherein the pH adjuster is a phosphate.

7. An emulsified preparation according to Claim 6, wherein the phosphate is dihydrogenphosphate or hydrogenphosphate.

8. An emulsified preparation according to Claim 7, wherein the dihydrogenphosphate is sodium dihydrogenphosphate or potassium dihydrogenphosphate.

9. An emulsified preparation according to Claim 7, wherein the hydrogenphosphate is sodium hydrogenphosphate or potassium hydrogenphosphate.

10. An emulsified preparation according to Claim 2, wherein the lower perfluoroalkyl group is a perfluoromethyl group.

11. An emulsified preparation according to any preceding claim wherein the particle size of the emulsified preparation is 0.05 to 0.3 µm.

12. An emulsified preparation according to any preceding Claim, wherein the aqueous solvent is distilled water for injection, sterile water, saccharide solution or electrolyte.

13. A method of preparing an emulsion of a perfluorocarbon, which method comprises admixing, in any

order, 5-50% (w/v) of a perfluorocarbon compund represented by the formula:

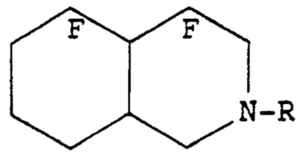

(wherein R represents a lower perfluoroalkyl group), 1-5% (w/v) of a phospholipid, 0.01-0.1% (w/v) of a fatty acid or an ester or a salt thereof, 0.001-0.01% (w/v) of tocopherol, 0.011-1.1% (w/v) of a pH adjuster and an adequate amount of an aqueous solvent, to form a crude emulsion and then homogenizing the crude emulsion.

14. A method according to claim 13, wherein the emulsion has a particle size of from 0.05 to 0.3 um inclusive.